# EUROPEAN PATENT APPLICATION

(11) **EP 1 281 773 A1**
(43) Date of publication of application: **05.02.2003**
(21) Application number: 01118402.5
(22) Date of filing: 31.07.2001
(51) Int. Cl.: C12Q 1/68

(54) **Method to detect splice variants of the calcineurin gene**

(71) Applicant: GENOPIA Biomedical GmbH, 66123 Saarbrücken (DE)
(72) Inventor: Völkel, Helge, Dr., 89081 Ulm (DE)
(74) Representative: Patentanwälte Ruff, Wilhelm, Beier, Dauster & Partner

(57) **Abstract**

A method for detecting isoforms and/or splice variants of Calcineurin or its subunits is established. The inventive method is especially useful for differentiation between the various isoforms of Calcineurin. Herein a polymerase chain reaction of nucleic acids of mammalian tissue is performed and the corresponding products are evaluated. Especially prefered is a reverse transcribed polymerase chain reaction. For the polymerase chain reaction primers for the isoforms and/or splice variants of Calcineurin are used. In a prefered embodiment isoforms and/or splice variants of the catalytic subunit Calcineurin-A are detected.

SEQUENCE LISTING

<110> GENOPIA Biomedical GmbH

<120> Detection of Calcineurin isoforms

<130> A 35445 EP

<140>
<141>

<160> 5

<170> PatentIn Ver. 2.1

<210> 1
<211> 23
<212> DNA
<213> Artificial Sequence

<220>
<223> Description of Artificial Sequence: primer for
      Calcineurin isoform

<400> 1
atctgctcag acgatgaact rgg                                          23

<210> 2
<211> 21
<212> DNA
<213> Artificial Sequence

<220>
<223> Description of Artificial Sequence: primer for
      Calcineurin isoform

<400> 2
ggcatcctct cgttaattcg g                                            21

<210> 3
<211> 23
<212> DNA
<213> Artificial Sequence

<220>
<223> Description of Artificial Sequence: primer for

Calcineurin isoform

<400> 3
atttgctctg atgatgaact aat                                              23


<210> 4
<211> 21
<212> DNA
<213> Artificial Sequence

<220>
<223> Description of Artificial Sequence: primer for
      Calcineurin isoform

<400> 4
ggcattctct cattgatcct a                                                21


<210> 5
<211> 23
<212> DNA
<213> Artificial Sequence

<220>
<223> Description of Artificial Sequence: primer for
      Calcineurin isoform

<400> 5
catatgctcg gatgaagaaa tga                                              23

## Description

The invention relates to a method for detection and analysis of Calcineurin isoforms and/or splice variants thereof and to diagnostic methods.

Calcineurin (E.C 3.1.3.16) is a serine/threonine phospo-protein phosphatase and is composed of a catalytic (Calcineurin A) and regulatory (Calcineurin B) subunit. The catalytic subunit is about 60 and the regulatory subunit is about 18 kDa. Calcineurin is the only protein phosphatase known to be under the control of calcium and calmodulin. Binding of calcium and calmodulin is necessary for enzymatic activity. Calmodulin is bound by the catalytic subunit whereas the regulatory subunit possesses four calcium binding sites.

Calcineurin is discussed in various contextes. For example Calcineurin has been implicated in neuronal signaling pathways (Klee et al., 1988; Jakel, 1997) but the neuronal function is only poorly understood (Guerini, 1997). Furthermore Calcineurin is discussed in the context of immunosuppression, wherein Calcineurin acts via the transcription factor NFAT (Nuclear factor of activated T-cells) on the T-cells response.

Besides these examples it is known, that Calcineurin has been implicated in a variety of diseases. Thus it has been shown that Calcineurin plays a key role in heart failure (e.g. Force et al., 1999; Izumo and Aoki, 1998; Lim and Molkentin, 1999) and stroke (Morioka et al., 1999). Furthermore Calcineurin has been implicated in the pathogenesis of amyotrophic lateral sclerosis (Ferri et al., 2000), skeletal muscles diseases (Chin et al., 1998) and epilepsy (Lie et al., 1998).

As mentioned above, Calcineurin is formed by two subunits. Both subunits, the catalytic subunit (Calcineurin A) and the regulatory subunit (Calcineurin B) occur in different isoforms which are encoded by different genes. In mammals, three distinct genes (A-α, A-β, A-γ) for the catalytic subunit have been characterized, each of which can undergo alternative splicing to yield additional variants. Especially these isoforms have been implicated in the diseases mentioned above. But up to now the specific role of the different isoforms in the development and maintenance of said diseases is not known. Nevertheless, a detection and analysis of Calcineurin isoforms is a very useful target for diagnosis of different cardiovascular diseases, epilepsy and neurodegenerative diseases, for example. Furthermore a reliable detection and analysis method for Calcineurin isoforms is a prerequisite for further research in these fields and other fields. In addition, for example stroke and minor cardiac failures are often unnoticed in the beginning, therefore biomarkers are also very important to start treatment in time and evaluate the prognosis of the diseases.

Up to now the existing methods for the detection of Calcineurin isoforms are based on immunoblotting techniques. These methods are not well-suited for research and especially diagnosis, since these methods are both time-consuming and expensive. Therefore, the invention has the object to provide a new method for the detection and analysis of Calcineurin isoforms. By the use of such a method it would be possible to get insight into the development and maintenance of different diseases which are known to be connected with different isoforms of Calcineurin. Furthermore, the method would provide a useful tool in diagnosis, especially in very early diagnosis of different diseases.

This object is solved by a method according to claim 1. Prefered embodiments of the inventive method are depicted in the dependent claims 2 to 10. Primers and a kit suitable for performing the inventive method are claimed in claims 11 to 17. Claims 18 to 21 refer to the use of said primers and claims 22 to 24 relate to a diagnostic method. The wording of all claims is hereby made to the content of the specification by reference.

In the inventive method for detection and analysis of isoforms and/or splice variants of calcineurin or its subunits polymerase chain reactions (PCR) are performed. For this step nucleic acids of mammalian tissue are used, special segments are amplified as PCR products and these products are evaluated. The inventive method is especially useful for differentiation between the various isoforms of Calcineurin.

The PCR is preferably a reverse transcribed PCR (RT-PCR). That means, that RNA of the tissue is extracted, especially total RNA. This RNA is reverse transcribed into cDNA by the use of oligo(dT) primers according to standard protocols. This cDNA is the template for the following PCR with specific primers for Calcineurin. The RT-PCR is especially prefered, since RNA reflects the actual status of the cell or the tissue, respectively. At the level of DNA all genes for the different isoforms of Calcineurin are present. It is the transcription of DNA into RNA, by which it is decided which isoform will be expressed in the cell or the tissue. Furthermore the splicing, which results into different splice variants of Calcineurin, takes place at the level of RNA. Therefore, the RNA is of special interest for the inventive method.

After the step of reverse transcription of the RNA into cDNA the PCR with primers specific for Calcineurin is performed. By this step the different isoforms and/or splice variants of Calcineurin are amplified.

Preferably said primers for the isoforms and/or splice variants used for PCR are not absolutely specific for Calcineurin isoforms of one species, but are essentially specific for Calcineurin isoforms. Said essentially specific primers are usefull in the amplification of Calcineurin of different species. By the use of these primers the inventive method is suitable for the analysis of Calcineurin of different species like human, mouse, rat, dog, cat etc. In an especially prefered embodiment of the inventive method the method is used for the analysis of Calcineurin of any mammalian tissue.

Especially prefered are primers for the isoforms and/or splice variants of the catalytic subunit Calcineurin-A (CN-A). By the use of these primers in the inventive manner said isoforms and/or splice variants of Calcineurin are analyzed. Preferably these isoforms are CN-Aα, CN-Aβ and/or CN-Aγ and/or their splice variants. As outlined above it has been shown that especially these isoforms are of clinical relevance. Nevertheless, the invention comprises the use of primers specific for isoforms and/or splice variants of the regulatory subunit also in order to analyze these forms of the regulatory subunit.

Advantageously the inventive primers are directed to the 3'-region of the relevant sequences, since this region is biologically better accessible than the 5'-region. Preferably the primer pairs embrace regions of potential splice variants of the isoforms. It is especially prefered that the primers are directed to regions of the isoforms, in which the isoforms are especially different.

In a prefered embodiment of the inventive method the following primers for the isoforms of the catalytic subunit and their splice variants are used.
The primers for CN-Aα are:

(SEQ ID NO. 1) atg tgc tca gac gat gaa ctr gg (CN-Aα-sense 1)
(SEQ ID NO. 2) ggc atc ctc tcg tta att cgg (CN-Aα-antisense 1).

The primers for CN-Aβ are:

(SEQ ID NO. 3) att tgc tct gat gat gaa cta at (CN-Aβ-sense 1)
(SEQ ID NO. 4) ggc att ctc tca ttg atc cta (CN-Aβ-antisense 1).

The primers for CN-Aγ are:

(SEQ ID NO. 5) cat atg ctc gga tga aga aat ga (CN-Aγ-sense 1)
(SEQ ID NO. 2) ggc atc ctc tcg tta att cgg (CN-Aγ-antisense 1).

In the listing of the primers the primers are written in 5'-3' direction. "a" means adenine, "t" means thymine, "g" means guanine and "c" means cytosine. "r" means "a" and "g". Primers CN-Aα-antisense 1 and CN-Aγ-antisense are identical.

The different isoforms and/or splice variants of calcineurin which are specific for several diseases as outlined above, are especially present in nervous tissue. Therefore in a prefered embodiment of the inventive method the mammalian tissue as source of nucleic acids is nervous tissue, especially central nervous tissue. Preferably brain tissue is used like amygdala, frontal cortex, hippocampus, piriform cortex, corpus striatum and/or spinal cord. Especially prefered is the analysis of tissue of both hemispheres of the brain in separate approaches.

In another prefered embodiment of the invention said mammalian tissue is cardiac tissue since Calcineurin isoforms play a key role in heat failure, for example.

According to the invention the PCR products are evaluated. This could be done by common methods which are known to an expert skilled in the art. In a prefered embodiment of the inventive method the PCR products are separated by electrophoresis and visualised by ethidium bromide staining. Obviously other separating and staining methods are also possible. In another embodiment of the inventive method the PCR products are separated and blotted onto a membrane. These blotted PCR products are detected by labeled probes, for example by radioactive labeled probes. The separation of PCR products is especially prefered in order to differentiate the isoforms of Calcineurin by differences in the molecular weight.

In another prefered embodiment of the invention the PCR products are detected without separation of the products. This is performed by treatment of the solution comprising the PCR products with a corresponding labeled probe and analyzing the interaction of PCR products and probe by adequate methods, for example. In this embodiment the separation step is spared and this is especially advantageous with regard to automation of the whole process.

In a prefered embodiment of the inventive method the evaluation of the PCR products is performed in comparison with an interal standard. A suited internal standard is always expressed in the same amount and is not influenced by the diverse conditions of the cell or the tissue. One example of a suited internal standard is actin. According to the inventive method a separate PCR is performed with a primer pair specific for actin. The resulting PCR product is analysed in parallel to the PCR products of Calcineurin and the amounts of the different products are compared.

Thereby it is possible to make quantitative assertions regarding the expression of the different isoforms and/or splice variants of Calcineurin.

In a prefered embodiment of the inventive method the resulting PCR products are at least partly sequenced. This is especially prefered in order to identify the PCR products and relate them to certain isoforms and/or splice variants of Calcineurin. Preferably the sequencing of the PCR products is performed by standard protocols known to an expert in the art.

Moreover the invention comprises primers for PCR with the following sequences:

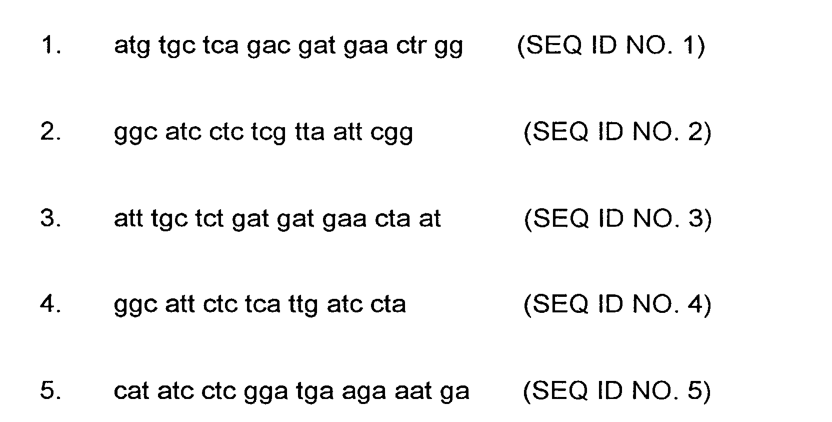

These primers are especially useful for performing PCR in order to obtain the amplification of different isoforms and/or splice variants of the catalytic subunit of Calcineurin. By the use of the primers 1. and 2. CN-Aα is obtained. By the use of the primers 3. and 4. CN-Aβ is obtained. By the use of the primers 5. and 2. CN-Aγ is obtained.

As template for PCR using these primer pairs generally nucleic acids extracted from any tissue are suited. Especially prefered are templates derived from mammalian tissues.

Furthermore the invention comprises a kit for performing a method for analysis of isoforms and/or splice variants of Calcineurin or its subunits. The most important ingredients of said kit are primers for isoforms and/or splice variants of a subunit, especially the catalytic subunit, of Calcineurin. In a prefered embodiment of this inventive kit the kit comprises different components for performing PCR and/or RT-PCR. Preferably these components are reagents for extraction of RNA and/or primers for making cDNA and/or primers for an internal standard, especially for actin, and/or reagents for performing PCR, for example buffers, salt solutions, nucleotides and water. Regarding the specific primers for isoforms and/or splice variants of Calcineurin reference is made to the description above.

Additionally the invention comprises the use of at least one of the primers above for a method for detecting and analysing isoforms and/or splice variants of Calcineurin or its subunits. In this embodiment it is especially prefered that the primer is placed on a DNA chip because such chips are especially suited for the use in automated processes. Furthermore the invention comprises the use of at least one PCR product obtained from a polymerase chain reaction using at least one of the primers described above. These PCR products are useful in a method for detecting and analysing isoforms and/or splice variants of Calcineurin or its subunits. Advantageously the primers and/or PCR products are used as hybridising probes in order to detect the respective forms of Calcineurin.

Finally the invention comprises a method for diagnosis of diseases and/or early recognition of potential diseases, in which Calcineurin is involved. Preferably these diseases are cardiovascular diseases, epilepsy and/or neurodegenerative diseases. This inventive method is characterized by performing PCR, especially RT-PCR, of nucleic acids of mammalian tissue and furthermore the evaluation of the PCR products obtained. Regarding further features of this method reference is made to the description above.

The described features and further features of the invention result in greater detail from the following examples, the figures and the subclaims. The diverse features could be realized alone or in combination with each other.

In the figures it is shown:

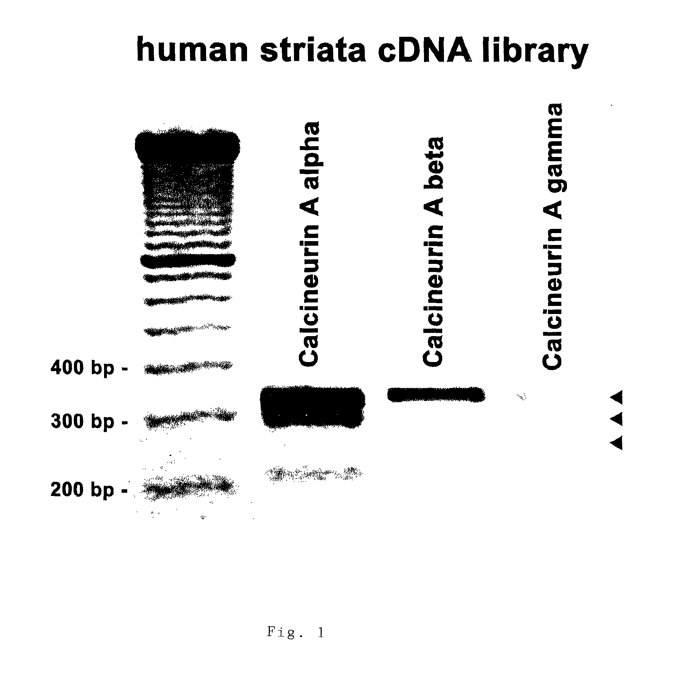

Figure 1: Expression of CN-A isoforms in human brain (human striatum cDNA library). Calcineurin-Aα showed three distinct bands, containing the 341 bp and 311 bp splice variants. The Calcineurin-Aβ isoform was only detected as a 341 bp splice variant. The calcineurin-Aγ isoform resulted in three different PCR fragments containing the 336 bp and 306 bp splice variants.

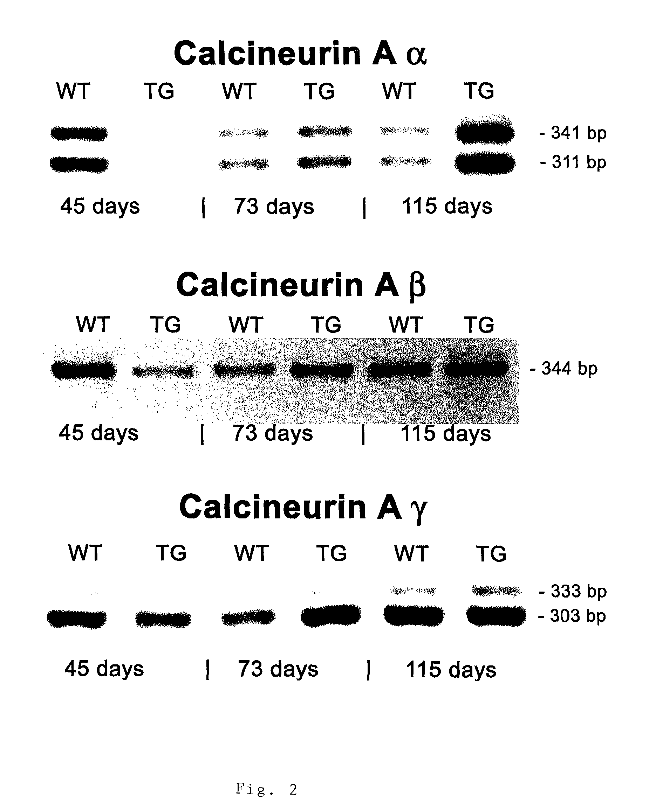

Figure 2: Expression of calcineurin isoforms in a transgenic mouse model for a neurodegenerative disease (amyotrophic lateral sclerosis, ALS). TG: transgenic (ALS), WT: wildtype control (no disease).

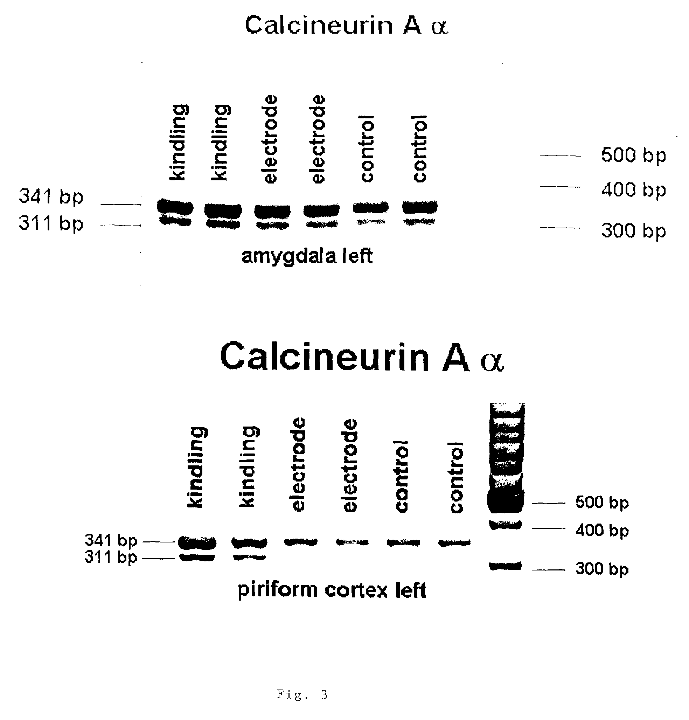

Figure 3: Calcineurin-Aα isoform expression in a model for epilepsy (kindled rats). In the upper panel it is shown the expression in amygdala whereas in the bottom panel insights in the situation in piriform cortex are given. Both panels reveal typical examples for differential expression in kindled animals. Lanes are named as follows:
   - kindling:: rats were transplanted with electrodes and stimulated
   - electrode:: sham operated with an electrode but not stimulated
   - control:: sham operated without any transplantation.

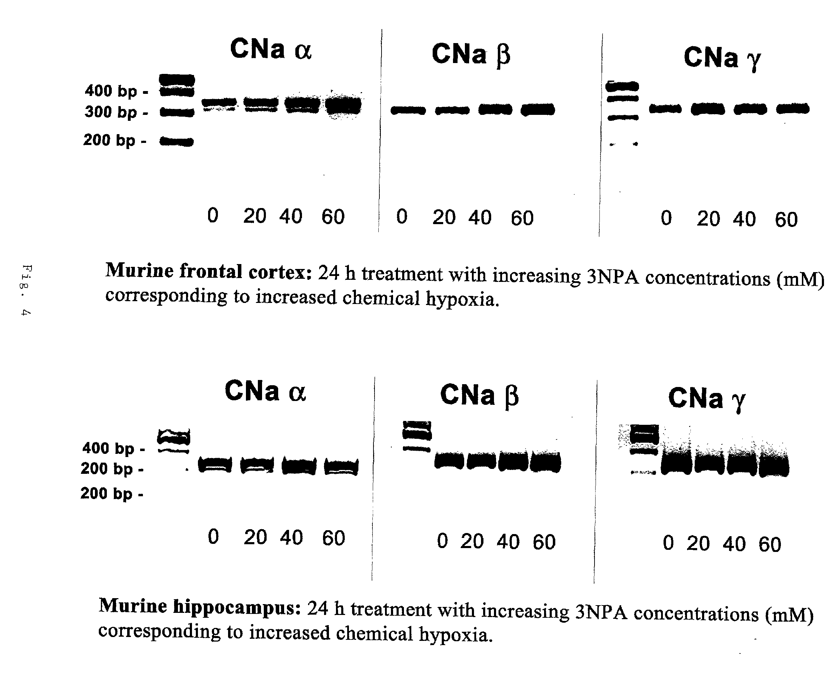

Figure 4: Calcineurin-A isoform expression in a model for cardiovascular diseases (hypoxic murine brain tissues slices). CN-Aα, CN-Aβ and CN-Aγ were induced in hypoxic murine cortex (upper picture). CN-Aα and CN-Aβ were induced in hypoxic murine hippocampus. CN-Aγ levels are constant in the range of 20 - 60 mM 3NPA-treatment (bottom picture).

### Examples

Extraction of RNA was done with Trizol™ reagent (GIBCO, Paisley, USA). 1 µg of total RNA was reverse transcribed with oligo(dT)primers according to standard protocols. From this reaction, 20 - 100 ng cDNA was used in a β-actin PCR for standardisation. The human cDNA was supplied by Stratagene (San Diego, USA). The β-actin-primer pairs were supplied by Promega (Heidelberg, Germany). β-actin PCRs were conducted in a 50 µl volume, containing 5 µl 10x PCR-buffer (500 mM KCI, 100 mM Tris HCI pH 9.0, 1%Triton X-100), 3µl 25 mM MgCl₂, 1µl 10mM dNTPs, 2.5 µl beta-actin sense primer and 2.5 µl beta-actin antisense primer, 0.5 to 2 µl diluted cDNA (20 - 100 ng) and RNA free water ad 50 µl.

A "hot start" PCR was done by addition of 2.5 units of Taq polymerase after a first heating step at 94°C for 1 min. The amplification conditions were 25 cycles of 30 sec. at 94°C for denaturing, 1 min. at 55°C for annealing and 2 min. at 68°C for elongation. PCR was terminated with a 10 min. extension step at 68°C. One fifth of the reactions was analysed by electrophoresis in an ethidium bromide stained visigel separation matrix from Stratagene. Quantification of DNA bands was performed with Multi-Analyst Software from Biorad (München, Germany) by comparing the intensity of Calcineurin with the beta-actin control containing equal amounts of cDNA. For the amplification of Calcineurin isoforms, the primer pairs as described above were used.

Calcineurin PCRs were conducted in a 50 µl volume. They contained 5 µl 10x PCR-buffer (500 mM KCI, 100 mM Tris HCI pH 9.0, 1%Triton X-100), 3 µl 25 mM MgCl₂, 1 µl 10 mM dNTP-mix, 2.5 µl sense primer, 2.5 µl antisense primer, the calculated amount of diluted cDNA (0.5 - 100 ng) and RNA free water ad 50 µl. "Hot start" PCR was done by addition of 2.5 units of Taq polymerase after a first heating step at 94°C for 1 min. The primer combinations and amplification conditions were as follows:
Calcineurin Aα: Primers 1. and 2. with 25 cycles of 30 sec 94°C, 1 min 60°C and 2 min 72°C followed by final extension step of 10 min 72°C.
Calcineurin Aβ: Primers 3. and 4. with 30 cycles of 30 sec 94°C, 1 min 55°C and 2 min 72°C followed by final extension step of 10 min 72°C.
Calcineurin Aγ: Primers 5. and 2. with 30 cycles of 30 sec 94°C, 1 min 55°C and 2 min 72°C followed by final extension step of 10 min 72°C.

One fifth of the reactions was analysed by electrophoresis in an ethidium bromide stained visigel separation matrix (Stratagene). Quantification of band intensity was performed with Multi-Analyst Software from Biorad. All calcineurin bands were sequenced to confirm the specificity of the primer sets.

Mice transgenic for a mutant superoxide dismutase 1 gene associated with familial amyotrophic lateral sclerosis were bred. After decapitation and preparation tissues were immediately frozen in liquid nitrogen. RNA was prepared from frozen tissue using Trizol™ reagent from GIBCO.

Hypoxic mice and respective controls were treated as described in Büchner et al. (1999), Neuroscience Letters 276: 131 - 134. After decapitation brain slices were immediately frozen in liquid nitrogen. RNA was prepared according to the protocols of transgenic mice.

Kindled rats were treated and prepared as published in Löscher et al. (1998), Exp. Neurol. 154: 551 - 559.

The above described methods were used to assay Calcineurin A isoform expression in three different mammalian species, i.e. mouse, rat and human and in six different tissues, i.e. amygdala, frontal cortex, hippocampus, piriform cortex, corpus striatum and spinal cord. As shown in figure 2, the bands obtained showed the expected sizes of 341 bp and 311 bp for the two Calcineurin-Aα splice variants (all species), 344 bp and 314 bp for the two Calcineurin-Aβ splice variants (rat and mouse; 341/311 bp for human), 333 bp and 303 bp for the Calcineurin-Aγ splice variants (mouse and rat; 336/306 bp for human) and 285 bp for the β-Actin (all species, not shown).

As example of neurodegenerative diseases a model for amyotrophic lateral sclerosis was analysed. Differential expression of Calcineurin was assayed in mice transgenic for a mutant superoxide dismutase 1 gene associated with familial amyotrophic lateral sclerosis (fig. 2). The transgenic mice (n=6) showed that at a time point clearly preceeding any pathological changes (45 d) the expression of CN-Aα, CN-Aβ and CN-Aγ were lower than in control animals (n=6). At day 73 (about the time when the first pathological changes as degradation of mitochondria or vacuolation of pericarya can be observed), CN-Aα expression is stronger than in wild types, whereas CN-Aβ and γ expression shows a level comparable to wild types. At day 115, when pathological changes can be seen but still before onset of pareses, the CN-Aα expression is slightly lower than controls, whereas CN-Aβ and CN-Aγ mRNA levels were still comparable to wild types.

As example for epilepsy kindled rats were analysed. Analyzing calcineurin isoforms in kindled rats revealed significant differences in the expression of CN-Aα in the left amygdala and the left part of the piriform cortex when compared with sham operated controls (transplanted electrode without stimulation; a typical example is shown in fig. 3). All together 144 examples were examined, i.e. 6 animals for each brain tissue, each hemisphere and each treatment group (kindling, electrode control, untreated control).

| tissue | isoform | kindling versus electrode control (significance)* | kindling versus electrode control (%) | kindling versus untreated control (significance)* | kindling versus untreated control (%) |
|---|---|---|---|---|---|
| Amygdata (left hemisphere) | CN-A-α 341 bp | p<0.01 | "+ 30.59 | p<0.01 | "+ 76.43 |
| Amygdala (left hemisphere) | CN-A-α 311 bp | P<0.01 | "-1.80 | p<0.01 | "+ 35.58 |
| Piriform cortex (left hemisphere) | CN-A-α 341 bp | p<0.01 | "- 0.83 | p<0.01 | "+ 116.2 |
| Piriform cortex (left hemisphere) | CN-A-α 311 bp | p<0.01 | "+ 22.12 | p<0.01 | "+ 264.96 |

As example of cardiovascular diseases hypoxic mice were analyzed. All calcineurin-A isoforms were detected in hypoxic mice brain (fig. 4). All Calcineurin-A isoforms, i.e. CN-Aα, CN-Aβ and CN-Aγ were induced in hypoxic murine cortex. In hypoxic murine hippocampus only CN-Aα and CN-Aβ were induced, whereas CN-Aγ levels were constant in a range of 0 - 30 mM 3NPA.

## Claims

1. Method for detecting isoforms and/or splice variants of Calcineurin or its subunits, **characterized in that** PCR (polymerase chain reaction), especially RT-PCR (reverse transcribed PCR), of nucleic acids of mammalian tissue is performed, and that the PCR products are evaluated.

2. Method according to claim 1, **characterized in that** primers for the isoforms and/or splice variants are used for PCR.

3. Method according to claim 1 or claim 2, **characterized in that** isoforms and/or splice variants of the catalytic subunit Calcineurin-A (CN-A) are detected.

4. Method according to one of the preceding claims, **characterized in that** the isoforms CN-Aα, CN-Aβ and/or CN-Aγ and/or their splice variants are detected.

5. Method according to one of claims 2 to 4, **characterized in that** said primers are for

CN-Aα (SEQ ID NO. 1): atg tgc tca gac gat gaa ctr gg and

(SEQ ID NO. 2): ggc atc ctc tcg tta att cgg,

CN-Aβ (SEQ ID NO. 3): att tgc tct gat gat gaa cta at and
(SEQ ID NO. 4): ggc att ctc tca ttg atc cta,

CN-Aγ (SEQ ID NO. 5): cat atg ctc gga tga aga aat ga and
(SEQ ID NO. 2): gc atc ctc tcg tta att cgg.

6. Method according to one of the preceding claims, **characterized in that** said mammalian tissue is nervous tissue, especially central nervous tissue, preferably brain tissue.

7. Method according to one of claims 1 to 5, **characterized in that** said mammalian tissue is cardiac tissue.

8. Method according to one of the preceding claims, **characterized in that** the PCR products are separated and/or made visible, especially by staining, preferably by ethidium bromide staining.

9. Method according to one of the preceding claims, **characterized in that** the evaluation is performed in comparison with an internal standard, especially with actin.

10. Method according to one of the preceding claims, **characterized in that** the PCR products are at least partly sequenced.

11. Primer for PCR with the sequence (SEQ ID NO. 1)

atg tgc tca gac gat gaa ctr gg

12. Primer for PCR with the sequence (SEQ ID NO. 2)

ggc atc ctc tcg tta att cgg

13. Primer for PCR with the sequence (SEQ ID NO. 3)

att tgc tct gat gat gaa cta at

14. Primer for PCR with the sequence (SEQ ID NO. 4)

ggc att ctc tca ttg atc cta

15. Primer for PCR with the sequence (SEQ ID NO. 5)

cat atg ctc gga tga aga aat ga

16. Kit, especially for performing a method according to one of the preceding claims, **characterized in that** it comprises
- primers for isoforms and/or splice variants of a subunit, especially the catalytic subunit, of Calcineurin
and preferably
- reagents for extraction of RNA and/or
- primers for making cDNA and/or
- primers for an internal standard, especially for actin, and/or
- reagents for performing PCR.

17. Kit according to claim 16, **characterized in that** it comprises at least one primer according to one of claims 11 to 15.

18. Use of at least one primer according to one of claims 11 to 15 for a method for detecting isoforms and/or splice variants of Calcineurin or its subunits.

19. Use according to claim 18, **characterized in that** said primer is placed on a DNA chip.

20. Use of at least one PCR product obtained from a polymerase chain reaction using at least one primer according to one of claims 11 to 15 for a method for detecting isoforms and/or splice variants of Calcineurin or its subunits.

21. Use according to claim 20, **characterized in that** said PCR product is placed on a DNA chip.

22. Method for diagnosis of diseases and/or early recognition of potential diseases, in which Calcineurin is involved, **characterized in that** PCR (polymerase chain reaction), especially RT-PCR (reverse transcribed PCR), of nucleic acids of mammalian tissue is performed, and that the PCR products are evaluated.

23. Method according to claim 22, **characterized in that** the diseases are cardiovascular diseases, epilepsy and/or neurodegenerative diseases.

24. Method according to claim 22 or claim 23, **characterized in that** at least one method according to one of claims 2 to 10 is performed.
